(19) Europäisches Patentamt
European Patent Office
Office européen des brevets

(11) Veröffentlichungsnummer: **0 272 530 B1**

(12) **EUROPÄISCHE PATENTSCHRIFT**

(45) Veröffentlichungstag der Patentschrift: **16.10.91**

(51) Int. Cl.⁵: **A61M 5/20, A61M 5/14**

(21) Anmeldenummer: **87118076.6**

(22) Anmeldetag: **07.12.87**

(54) **Gerät zur Applikation flüssiger Systeme.**

(30) Priorität: **18.12.86 CH 5021/86**

(43) Veröffentlichungstag der Anmeldung:
**29.06.88 Patentblatt 88/26**

(45) Bekanntmachung des Hinweises auf die
Patenterteilung:
**16.10.91 Patentblatt 91/42**

(84) Benannte Vertragsstaaten:
**AT BE CH DE FR GB IT LI LU NL SE**

(56) Entgegenhaltungen:
| | |
|---|---|
| EP-A- 0 103 073 | EP-A- 0 168 675 |
| EP-A- 0 195 637 | GB-A- 207 710 |
| US-A- 4 316 463 | US-A- 4 340 048 |
| US-A- 4 552 561 | |

(73) Patentinhaber: **F. HOFFMANN-LA ROCHE AG**
**Postfach 3255**
**CH-4002 Basel(CH)**

(72) Erfinder: **Cirelli, Giorgio, Dr.**
**Kirchweg 41b**
**CH-5415 Nussbaumen(CH)**
Erfinder: **Steffen, Hans, Dr.**
**Seestrasse 16**
**CH-4410 Liestal(CH)**
Erfinder: **Surber, Christian, Dr.**
**Gellertstrasse 11**
**CH-4052 Basel(CH)**

(74) Vertreter: **Körber, Wolfhart, Dr.rer.nat. et al**
**Patentanwälte Dipl.-Ing. H. Mitscherlich**
**Dipl.-Ing. K. Gunschmann Dr.rer.nat. W. Kör-**
**ber Dipl.Ing. J. Schmidt-Evers Dipl.-Ing. W.**
**Melzer Steinsdorfstrasse 10**
**W-8000 München 22(DE)**

## Beschreibung

Die Erfindung betrifft ein tragbares Gerät zur intradermalen oder subkutanen Injektion einer flüssigen Wirkstoffformulierung mit einem Vorratsbehälter für die Wirkstoffformulierung, einer mit dem Behälter in Verbindung bringbare Injektionsnadel, einer Pumpeneinrichtung zum Entleeren des Behälters durch die Injektionsnadel und Befestigungsmitteln zum Befestigen des Geräts an einer geeigneten Körperstelle des Patienten.

Seit einiger Zeit zeichnet sich deutlich das Bedürfnis nach kleinen, kompakten und tragbaren Injektionsvorrichtungen ab, die auf einer geeigneten Körperstelle getragen werden und die während längerer Zeitspannen genau definierte Mengen von Wirkstoffen in den Körper, vorzugsweise subkutan, abgeben. Es sind schon verschiedene Vorrichtungen dieser Art bekannt, die gegenüber der konventionellen Subkutanspritze erhebliche Vorteile aufweisen. Bei der herkömmlichen subkutanen Injektion wird ein sogenannter Bolus eines Wirkstoffs in den Körper gebracht und muss dort allmählich absorbiert und im Körper verteilt werden. Diese Verteilung hängt stark von den physiologischen Gegebenheiten des Individuums ab und ist daher nicht mehr kontrollierbar. Vorrichtungen, die einen Wirkstoff über längere Zeit hinweg kontinuierlich abgeben haben demgegenüber den Vorteil, dass eine genau auf die Bedürfnisse des Körpers abgestimmte Kontrolle möglich ist. Es ist sogar möglich, Phasen der Wirkstoffabgabe mit Phasen, in denen kein Wirkstoff abgegeben wird, abzuwechseln. Speziell bei hochwirksamen Wirkstoffen, wie beispielsweise Insulin, Interferon und dergleichen ist eine derart physiologisch angepasste Wirkstoffabgabe vorteilhaft.

Bekannte Geräte dieser Art haben üblicherweise einen Behälter für den Wirkstoff, mit dem eine Injektionsnadel verbunden ist. Der Behälterinhalt wird durch eine Pumpvorrichtung über eine vorbestimmte Zeitspanne hinweg verteilt in den Körper abgegeben. Ein Beispiel einer solchen Vorrichtung ist in DE 3121888 beschrieben. Das Gerät enthält einen Vorratsbehälter in Form eines Schlauches, der durch eine von einem Uhrwerk angetriebene Quetschrolle durch eine am Schlauchende angeschlossene Injektionsnadel entleert wird. Die Injektionsnadel wird in einiger Entfernung vom Gerät in dar Hautgewebe eingestochen. Das Gerät ist ähnlich wie eine Armbanduhr am Handgelenk zu tragen. Eine andere Lösung ist im U.S. Patent 4,552,561 beschrieben. Dieses Gerät ist auf der Haut aufklebbar und trägt die Injektionsnadel auf seiner Unterseite, so dass die Einstichstelle während der Anwendung vom Gerät abgedeckt wird. Auch bei diesem Gerät muss die Nadel in das Hautgewebe eingestochen werden. Sowohl bei den herkömmlichen subkutanen Bolusinjektionen, als auch bei den erwähnten neueren subkutanen Injektionsvorrichtungen ist vor allem das Einstechen der Injektionsnadel problematisch. Medizinische Laien haben meistens keine ausreichende Uebung eine solche Nadel richtig zu plazieren und eine häufig durchaus berechtigte Hemmung wegen des zu erwartenden Schmerzes.

Der Erfindung liegt die Aufgabe zugrunde. eine tragbare Injektionsvorrichtung der erwähnten Art bereitzustellen, bei dem die mit bekannten Geräten verbundenen Nachteile nicht auftreten und das insbesondere vom medizinischen Laien ohne weiteres angewendet werden kann.

Erfindungsgemäss wird dies erreicht durch ein Gerät der eingangs erwähnten Art, das sich auszeichnet durch eine Nadelantriebsvorrichtung zum Einschiessen der Injektionsnadel in die Haut des Patienten.

Das Gerät kann zusätzlich eine Flusskontrolleinrichtung enthalten, mit der die Wirkstoffabgabe entweder konstant gehalten oder gemäss einem bestimmten Profil vorgenommen werden kann.

Gemäss einer Weiterbildung der Erfindung besteht das Gerät aus zwei Teilen, von denen ein Teil mit den wertvolleren Elementen mehrfach benutzt und der andere Teil als Verbrauchsmaterial nach einmaliger Benutzung weggeworfen werden kann.

Als Behälter für den Wirkstoff bzw. die Wirkstofflösung kann beispielsweise einfach ein Teil des Gerätegehäuses vorgesehen werden, wobei durch eine geeignete Membran aus Elastomeren, Metallen und dergleichen in ein- oder mehrschichtiger Ausführung eine Kammer abgetrennt ist. Eine weitere Möglichkeit für einen Vorratsbehälter ist ein geschlossener Behälter wiederum aus Elastomeren, Metallen und dergl., beispielsweise in Form eines Balgs. Eine andere Lösung für den Vorratsbehälter ist ein Quetschschlauch, wie er beispielsweise in der einleitend erwähnten Patentbeschreibung gezeigt ist. Noch eine andere Möglichkeit stellt eine Kolbenspritze dar, deren Zylinder als Vorratsbehälter dient. Schliesslich kann auch noch ein saugfähiges Material ähnlich einem Schwamm, für die Wirkstoffaufnahme dienen. Es können mehrere voneinander getrennte Vorratsbehälter oder -kammern vorgesehen werden, beispielsweise im Fall der Injektion zweier Wirkstoffe oder bei Einsatz eines Wirkstofflyophilisats und einem Rekonstitutionslösungsmittel. Für den Antrieb der Pumpeinrichtung zum Entleeren des Behälters kommen unterschiedliche Energiequellen in Frage. So kann die Pumpe beispielsweise gasbetrieben sein, wobei der zum Entleeren des Behälters erforderliche Druck durch Elektrolyse-, Photolyse- oder chemische Reaktionen und schliesslich auch durch Treibdämpfe, wie z.B. Freon etc., erzeugt werden kann. Eine weitere Möglichkeit ist die Erzeugung des erforderlichen Druckes durch einfache Osmose oder Elektroosmose. Daneben sind selbstverständlich auch mechanische Antriebe geeignet, beispielsweise durch Fadern, Bimetalle, Gedächtnislegierungen oder auch durch Uhrentriebwerke. Schliesslich kommen auch elektrische oder magnetische Antriebe in Frage in Form der bekannten Elektropumpen, Membranpumpen, piezoelektrischen Pumpen, elektrischen Uhrentriebwerke oder Magneten.

Für die Befestigung des Geräts an der geeigneten Körperstelle des Patienten eignet sich vor allem eine Klebeschicht auf der Auflagefläche des Geräts, die sich gegebenenfalls in Form eines Pflasters über das Gerät hinaus erstreckt. Eine Alternative dazu ist beispielsweise ein Befestigungsband wie das in der eingangs erwähnten deutschen Offenlegungsschrift gezeigte Armband.

Als Antriebsvorrichtung für die Nadel wird bevorzugt eine Metallfeder vorgesehen.

Bei konstantem Fluss wird die konstante Freisetzung des Wirkstoffs z.B. mittels Kapillaren, Fritten oder Membranen eingestellt. Zusätzlich kann der Fluss durch Anpassung der Viskosität der flüssigen Wirkstofformulierung eingestellt werden.

Die Kontrolle der Abgabemenge des Wirkstoffs kann ebenfalls auf unterschiedliche Art verwirklicht werden. So kann beispielsweise zwischen dem Vorratsbehälter und der Nadel ein Quetschschlauch angeordnet sein, dessen Durchmesser je nach Bedarf erweitert oder reduziert wird. Eine weitere Möglichkeit besteht im Vorsehen eines Druckreduzierventils bekannter Art. Eine andere Möglichkeit besteht in der Auswahl eines bestimmten Bereichs der Ausdehnung der Antriebsfeder oder in der Benutzung besonderer Tellerfedern, bei denen die Kraft innerhalb vorgegebener Grenzen über eine Wegstrecke konstant ist. Eine aufwendigere Flusskontrolle ist auch durch Rückkopplung mittels Sensoren möglich. Schliesslich kann auch eine Flusskontrolle dadurch stattfinden, dass zum Antrieb der Behälterentleerung ein speziell programmiertes Pumpwerk eingesetzt wird.

Als Injektionsnadeln werden möglichst dünne Kapillaren, d.h. mit einem Durchmesser vorzugsweise < 0,5 mm, verwendet, da der Schmerz mit der Nadeldicke zunimmt. Auch die Einstichtiefe und der Schliff der Nadel wirken sich auf die Schmerzentstehung aus. Deshalb wird eine Einstichtiefe von 5000 µm mit Vorteil nicht überschritten. Ausserdem ist die Nadel vorzugsweise schräg angeschliffen, z.B. als Lanzettenschliff.

Wenn aufgrund des Nadeldurchmessers und der Einstichtiefe Schmerz vermieden wird, so kann doch durch die Wirkstofformulierung selbst ein Schmerz oder eine Reizung verursacht werden. Dem kann durch ein Lokalanesthetikum in der Wirkstofformulierung entgegengewirkt werden.

Im folgenden werden anhand der beiliegenden Zeichnungen Ausführungsbeispiele der Erfindung beschrieben.

Es zeigen

Fig. 1    einen Querschnitt durch eine Injektionsvorrichtung nach der Erfindung,

Fig. 2    eine Aufsicht auf die Vorrichtung nach Fig. 1 ohne Sicherungskappe 14

Fig. 3    einen Querschnitt durch eine andere Auführungsform der Erfindung

Fig. 4    eine Aufsicht mit teilweiser Schnittdarstellung der Vorrichtung nach Fig. 3,

Fig. 5    einen Querschnitt durch eine weitere Auführungsform der Erfindung,

Fig. 6    einen Schnitt entlang der Ebene B-B Vorrichtung nach Fig. 5.

Fig. 7    einen Querschnitt durch eine weitere Auführungsform der Erfindung.

Fig. 8    einen Schnitt entlang der Ebene A-A der Vorrichtung nach Fig. 7.

Fig. 9    einen Schnitt entlang einer Ebene senkrecht zur Achse einer weiteren Ausführungsform der Erfindung.

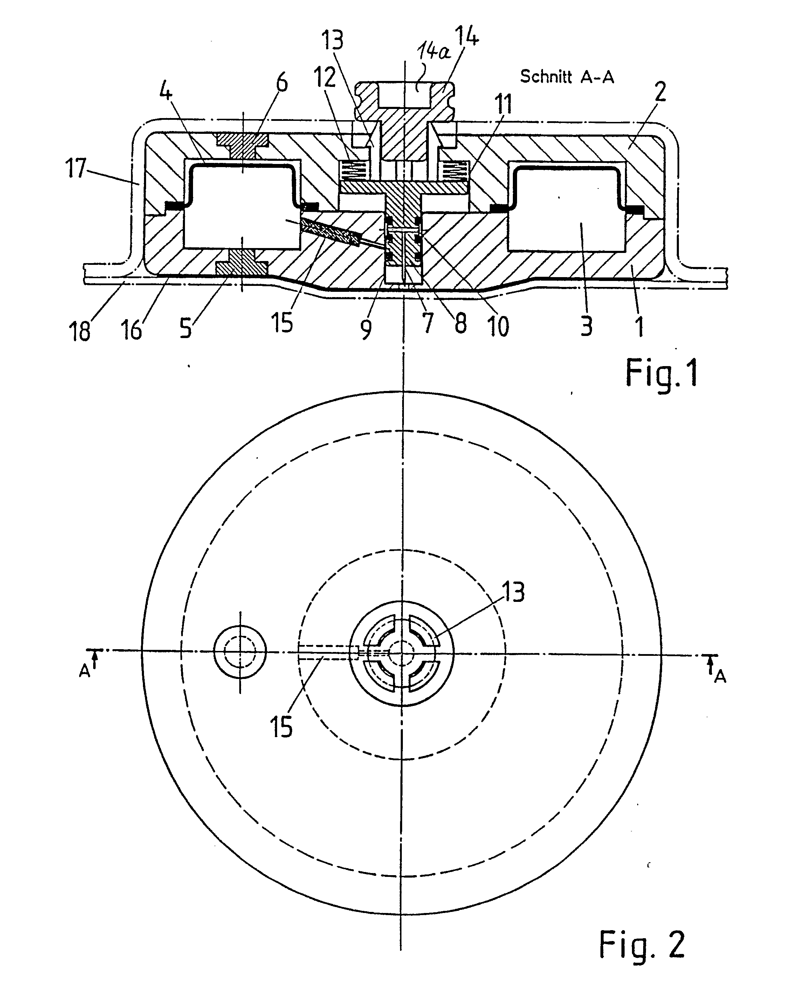

Die in den Figuren 1 und 2 gezeigte Vorrichtung besteht aus einem zweiteiligen flachzylindrischen Gehäuse mit einem Unterteil 1 und einem Oberteil 2. Die beiden Gehäuseteile sind fest, z.B. durch ein (nicht gezeigtes) Gewinde, miteinander verbunden. Durch ringförmige Ausnehmungen in den einander berührenden Flächen der beiden Teile wird eine ringförmige Kammer 3 gebildet. Durch eine Membran 4 wird die ringförmige Kammer in zwei voneinander getrennte Räume unterteilt. Beide Räume sind durch normalerweise mit Stopfen verschlossenen Einfüllöffnungen 5 und 6 mit der Aussenseite verbunden.

Im Zentrum des scheibenförmigen Gehäuses befindet sich eine Injektionsnadel 7 mit einer zu deren Einschiessen geeigneten Antriebsvorrichtung. Die Injektionsnadel besteht aus einer Stahlkapillare mit einem Durchmesser von 200 µm. Alternativ wäre gegebenenfalls eine Glaskapillare denkbar. Die Injektionsnadel 7 wird von einem Nadelträger 8 gehalten. Der Nadelträger 8 besitzt einen unteren zylindrischen Teil und einen oberen flachen scheibenförmigen Teil. Der zylindrische Teil ist in einer entsprechende Bohrung 9 des Gehäuseunterteils 1 axial verschiebbar angeordnet. Er besitzt eine quer zu seiner Achse geführte Bohrung 10 die mit dem Inneren der Hohlnadel 7 in Verbindung steht. Seine zylindrische Aussenfläche ist mit drei umlaufenden Nuten versehen, in die dichtende O-Ringe eingesetzt sind. Ferner befindet sich auf der Höhe der Bohrung 10 ebenfalls eine flache umlaufende Nut.

Die Bohrung 9 ist in ihrem untersten Teil auf einen Durchmesser verkleinert, der lediglich den Durchtritt der Nadel zulässt.

Der obere scheibenförmige Teil des Nadelträgers 8 ist in einer entsprechenden weiteren konzentrischen Bohrung 11 des Gehäuseoberteils 2 axial verschiebbar angeordnet. Auf etwa zwei Drittel der Dicke des Gehäuseoberteils ist die Bohrung 11 auf etwa ihren halben Durchmesser verkleinert. Dadurch wird ein Anschlag gebildet. Zwischen diesem Anschlag und dem Oberteil des Nadelträgers 8 ist eine Antriebsfeder 12 angeordnet, die dem Einschiessen der Nadel in die Haut des Patienten dient.

Der Nadelträger 8 ist ferner mit federnden Haltefingern 13 versehen, die sich von seiner Oberfläche nach oben erstrecken. Diese Haltefinger sind mit einem klinkenähnlichem Absatz versehen, der an der Kante einer Erweiterung der Bohrung 11 einrastet. Wenn die Haltefinger 13 eingerastet sind, befindet sich der Nadelträger 8 mit der Nadel 7 in seiner oberen Position, in der die Feder 12 gespannt ist und die Nadel nicht über die untere Fläche des Gehäuses hinausragt.

Zwischen den Haltefingern befindet sich eine Sicherungskappe 14, die verhindert, dass die federnden Haltefinger 13 unbeabsichtigt zusammengedrückt werden können und dadurch der Nadelantrieb ausgelöst wird. Auf der anderen Seite besitzt die Kappe eine Bohrung 14a, deren Durchmesser bei umgekehrtem Aufdrücken der Sicherungskappe 14 derart auf geeignete Schrägflächen der Haltefinger 13 passt, dass diese durch Aufdrücken der Kappe zusammengepresst werden und dadurch der Haltemechanismus ausgelöst wird.

Im Gehäuseunterteil ist eine Verbindungsbohrung 15 zwischen dem unteren Raum der Kammer 3 und der Bohrung 9 angeordnet. Diese Bohrung ist auf einem Teil ihrer Länge erweitert zur Aufnahme eines Drosselkörpers, der beispielsweise aus einer Teflonfritte besteht. Die Bohrung 15 ist so geführt, dass sie auf einer Höhe in die Bohrung 9 mündet, die der Bohrung 10 im Nadelträger entspricht, wenn dieser sich auf seiner unteren Position befindet. In der oberen Endlage ist die Mündung der Bohrung 15 in der Bohrung 9 durch den zylindrischen Teil des Nadelträgers 8 und die beiden unteren O-Ringe geschlossen.

Die Vorrichtung weist auf ihrer Unterseite eine Klebeschicht 16 auf. Ausserdem ist die Vorrichtung in ein entsprechend geformtes Befestigungspflaster 17 eingebettet. Die Klebeschicht 16 sowie auch die Klebeschicht des Pflasters sind vor dem Gebrauch durch eine Schutzfolie 18 abgedeckt. Die Klebeschicht 16 auf der Unterseite und diejenige des Pflasters können zusätzliche Wirkstoffe, beispielsweise ein Lokalanaesthetikum enthalten.

Bei die Herstellung wird nach dem Zusammenbau der Vorrichtung der untere raum der Kammer 3 über die Verbindungsöffnung 5 mit einem gewünschten Wirkstoff gefüllt und die Verbindungsöffnung 5 danach verschlossen. Ebenso wird der obere Raum der Kammer 3 durch die Verbindungsöffnung 6 mit einem Treibmittel gefüllt. Diese Füllungen sind in den meisten Fällen Herstellungsschritte und erfolgen dann nicht durch den Anwender. Im Fall von bestimmten Wirkstoffen kann es aber auch zweckmässig sein, die Füllung kurz vor der Anwendung vorzunehmen. Danach ist die Vorrichtung zur Anwendung bereit.

Bei der Anwendung ist die Funktionsweise wie folgt:

Der Benutzer zieht die Schutzfolie 18 ab und klebt die Vorrichtung mit Hilfe des Befestigungspflasters 17 und der Klebefolie 16 an einer geeigneten Körperstelle auf. Danach entfernt er die Sicherungskappe 14, dreht sie um und drückt sie auf die Schrägflächen der Befestigungsfinger 13. Diese werden auf diese Weise zusammengepresst und geben den Nadelantrieb frei. Die Feder 12 entspannt sich und drückt den Nadelträger 8 mit der Nadel 7 um die vorgesehene Distanz nach unten durch die Klebefolie hindurch in die Haut des Benutzers. Die Nadel soll etwa mindestens 50 µm und maximal etwa 5000 µm in die Haut eindringen. Wegen der geringen Eindringtiefe, des kleinen Durchmesser und des steilen Anschliffs der Nadel ist der Einstich schmerzlos oder schmerzarm.

Gleichzeitig wird mit der Verschiebung der Nadelträgers nach unten die Querbohrung 10 auf die Höhe der Mündung der Verbindungsbohrung 15 gebracht, so dass die Verbindungsbohrung nun mit der Nadel 7 in Verbindung steht. Der Wirkstofffluss durch die Nadel ist freigegeben und bestimmt sich einerseits durch den Druck des Treibmittels im oberen Raum der Kammer 3 und andererseits durch die Drosselwirkung des Drosselkörpers 15. Durch geeignete Auswahl dieser Fakoren, kann die Grösse des Wirkstoffstroms und damit die Dauer der Injektion bestimmt werden.

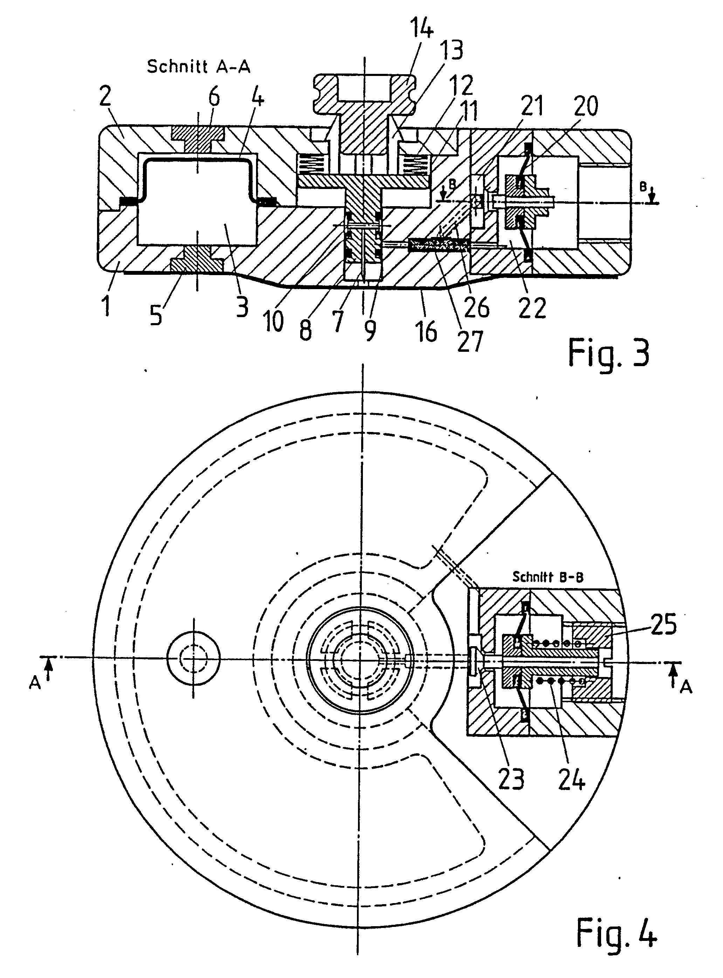

Die in den Figuren 3 und 4 gezeigte Vorrichtung ist bezüglich des Wirkstoffspeichers und bezüglich der Einschiessvorrichtung für die Nadel gleich aufgebaut wie das vorher beschriebenen Ausführungsbeispiel. Zur Kompensation von Druckschwankungen des Treibmittels ist beim vorliegenden Ausführungsbeispiel zusätzlich ein Druckreduzierventil (Druckbegrenzungsventil) vorgesehen. Das Druckreduzierventil ist in einem Segment des kreisförmigen Gehäuses angeordnet. Dementsprechend erstreckt sich die Kammer 3 nur über einen Teil des Umfangs.

Das Reduzierventil besitzt ein eigenes Gehäuse, das in eine entsprechende Aussparung des Gehäuses der Vorrichtung eingesetzt ist. Im Inneren besitzt es die von Druckreduzierventilen her bekannte, durch eine Membran 20 vorgenommene Unterteilung in eine Hochdruckkammer 21 und eine Niederdruckkammer 22. In der Verbindungsbohrung zwischen Hochdruckkammer 21 und Niederdruckkammer 22 ist ein Stempel 23 angeordnet, der im Zentrum der Membran 20 befestigt ist und durch axiale Verschiebung die Verbindungsöffnung verschliessen kann. Auf der anderen Seite der Membran befindet sich zwischen Stempel 23 und einem einstellbaren Wiederlager 25 eine Feder 24. Diese Feder bestimmt im wesentlichen den Druck, der in der Niederdruckkammer besteht. Die Hochdruckkammer 21 ist durch eine Verbindungsbohrung 26 mit dem unteren Raum der Kammer 3, d.h. dem Wirkstoffreservoir verbunden. Die Niederdruckkammer ist durch eine Bohrung 27 mit der zentralen Bohrung 9 verbunden und mündet wie beim vorher beschriebenen Beispiel auf der Höhe der Querbohrung 10, wenn sich der Nadelträger auf seiner unteren Endposition befindet.

Das Druckreduzierventil ermöglicht die Aufrechterhaltung einer weitgehend konstanten Abgaberate des Wirkstoffs, unabhängig von Druckschwankungen auf der Hochdruckseite. Solche Druckschwankungen können durch Aenderungen des Dampfdrucks des Antriebsmittels als Folge von Temperaturschwankungen auftreten.

Wie im vorher beschriebenen Beispiel wird diese Vorrichtung ebenfalls mit Klebeschicht, Befestigungspflaster und Schutzfolie versehen, und die Funktionsweise der Vorrichtung bei der Anwendung unterscheidet sich praktisch nicht von der beim vorher beschriebenen Beispiel.

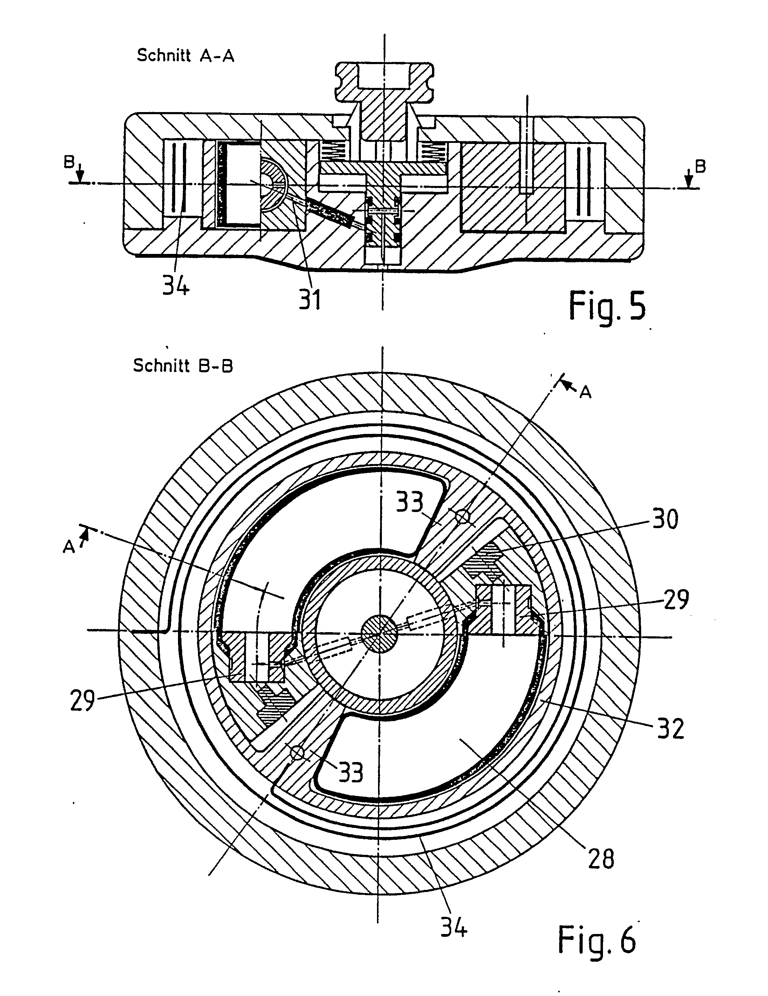

Bei der in den Figuren 5 und 6 gezeigten Vorrichtung ist eine alternative Vorrichtung zur Förderung des Wirkstoffs und eine andere Form des Reservoirs vorgesehen. Als Reservoir sind zwei Beutel, Blasen oder Bälge 28 vorgesehen, die auf einer Seite eine Oeffnung aufweisen, mit der sie an einer Halterung 29 befestigt sind, die auch die Füllöffnung 30 und die Verbindungsbohrung 31 zur zentralen Bohrung 9 aufweist. Die beiden Halterungen 29 sind fest mit dem zentralen zylindrischen Teil des Gehäuseunterteils 1 verbunden. Um diesen festen Teil herum ist ein Drehkörper 32 angeordnet, der zwei nach innen ragende Stege 33 aufweist, die jeweils an den Rückseiten der Reservoirbeutel 28 anleigen.

Zwischen dem Drehkörper und der Aussenwand des Gehäuseoberteils 2 ist ein Raum, in dem eine Spiralfeder 34 angeordnet ist. Diese Spiralfeder ist mit ihrem einen Ende in der Gehäusewand, mit ihrem anderen Ende im Drehkörper befestigt. Wenn sich die gespannte Spiralfeder entspannt, bewirkt sie ein Drehen des Drehkörpers, die mit den Stegen 33 die Reservoirbeutel 28 zusammendrückt und auf diese Weise den in ihnen enthaltenen Wirkstoff durch die Verbindungsöffnungen 31 entleert.

Durch Ausnutzung eines Teils der nutzbaren Windungszahl der Spiralfeder für die Drehbewegungen wird eine Steuerwirkung erreicht. Auf diese Weise erfolgt also die Förderung des Wirkstoffs und die Steuerung bzw. Kontrolle des Flusses gleichzeitig mit Hilfe der Antriebsfeder. Im übrigen entspricht die Funktion dieser Ausführungsform ebenfalls der in Figur 1 und 2 gezeigten Ausführungsform.

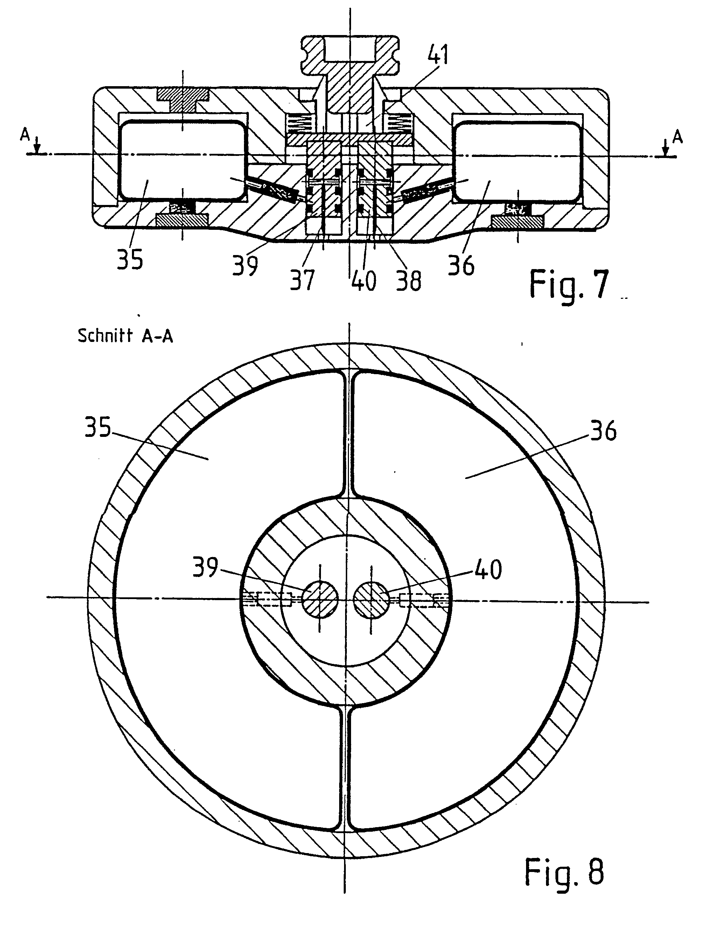

Die in den Figuren 7 und 8 gezeigte Vorrichtung besitzt in Abweichung von den bisher beschriebenen Ausführungsformen zwei Vorratsbehälter oder -kammern 35,36 und zwei separate mit Nadeln 37,38 versehene Nadelträger 39,40, die von einem gemeinsamen Betätigungsmechanismus 41 angetrieben werden. Diese Ausführungsform eignet sich für den Fall, dass zwei Wirkstoffe gleichzeitig und getrennt injiziert werden müssen.

Alternativ zu der gezeigten Version könnte anstelle der beiden Nadelträger mit zwei Nadeln auch ein einziger Nadelträger mit zwei Nadeln vorgesehen werden. Hierfür wäre ein aufwendigeres Dichtungssystem nötig.

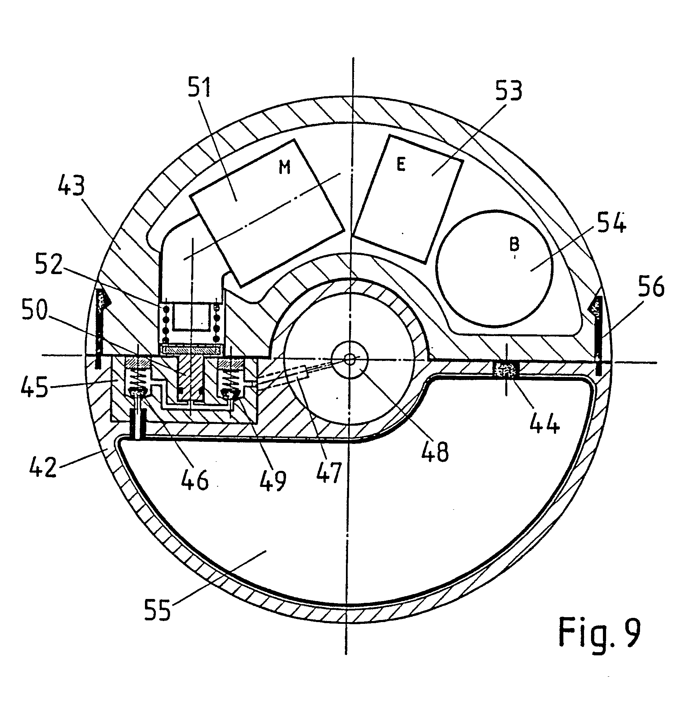

Das in Fig. 9 im Schnitt gezeigte Ausführungsbeispiel besitzt eine elektrisch angetriebene Wirkstofförderung. Wegen des verhältnismässig hohen Werts der elektrischen Elemente bei dieser Ausführungsform ist die Vorrichtung in einen Wegwerfteil 42 und einen wiederverwendbaren Teil 43 aufgeteilt. Die beiden Teile sind durch federnde Klammern 56, die in entsprechende Ausnehmungen eingreifen, miteinander verbunden.

Der Wegwerfteil 42 enthält wiederum einen Vorratsbehälter 55 mit einer durch ein Septum 44 verschlossenen Einfüllmöglichkeit. Durch das System kann das Reservoir ggf. erst kurz vor dem Gebrauch gefüllt werden. Ausserdem enthält der Wegwerfteil eine Pumpeinrichtung 45 mit einem mit dem Vorratsbehälter in Verbindung stehenden Ansaugventil 46, einem die Zuführleitung 47 zur Injektionsnadel 48 kontrollierenden Ausstossventil 49 und einer zwischen den beiden Ventilen angeordneten und mit beiden verbundenen Kolbenpumpe 50. Die Funktion der Pumpeinrichtung 45 ist wie folgt: Wenn der Kolben der Pumpe 50 aus der gezeigten Position zurückgezogen wird, wird durch das Ansaugventil 46 ein bestimmtes Volumen aus dem Vorratsbehälter angesaugt. Während dieser Phase ist das Ausstossventil 49 geschlossen. Wenn der kolben anschliessend wieder vorwärts bewegt wird, schliesst sich das Ansaugventil 46 und öffnet sich das Ausstossventil 49, so dass das angesaugte Volumen der Injektionsnadel zugeführt wird.

Die Injektionsnadel mit Nadelträger und Einstechmechanismus (hier nicht gezeigt) gehört ebenfalls zum Wegwerfteil.

Der wiederverwendbare wertvollere Teil 43 enthält den elektrischen Antrieb für die Pumpeinrichtung. Dieser besteht aus einem Elektromagneten 51, der das Zurückziehen des Kolbens der Pumpe 50 bewirkt, während die Vorwärtsbewegung durch eine Rückstellfeder 52 erfolgt. Der Elektromagnet 51 wird durch eine Steuerelektronik 52 gesteuert. Der Elektromagnet 51 und die Steuerung 53 werden von einer Batterie 54 mit Strom versorgt.

Eine elektrisch angetriebene Pumpe eignet sich besonders auch für die Kombination mit einer elektronischen Steuerung der Abgabemenge. Das Abgabeprofil kann z.B. vorprogrammiert und geeignet gespeichert sein. Die Kontrolle der Abgaberate kann durch geeignete Sensoren erfolgen.

Bei den gezeigten Ausführungsbeispielen ist die Nadelantriebsvorrichtung zum Einschiessen der Nadel mit der Funktion des Hahnes kombiniert. Es ist ohne weiteres möglich diese beiden Funktionen zu trennen, sodass z.B. zunächst nur die Nadel eingeschossen wird und dann separat ein Hahn geöffnet wird. Es sind auch andere Einstichbewegungen anstelle des einfachen zur Hautoberfläche senkrechten Einschiessens möglich. Beispielsweise kann die senkrechte Einstichbewegung mit einer Drehbewegung der Nadel gekoppelt oder die Nadel schräg zur Hautoberfläche eingeschossen werden.

**Patentansprüche**

1. Tragbares Gerät zur subkutanen oder intradermalen Injektion einer flüssigen Wirkstofformulierung mit einem Vorratsbehälter (3) für die Wirkstofformulierung, einer mit dem Behälter in Verbindung bringbaren Injektionsnadel (7), einer Pumpeinrichtung (4,32,34,50) zum Entleeren des Behälters durch die Injektionsnadel und Befestigungsmitteln (16,17) zum Befestigen des Geräts an einer geeigneten Körperstelle des Patienten, gekennzeichnet durch eine Nadelantriebsvorrichtung (12-13) zum Einschiessen der Injektionsnadel in die Haut des Patienten.

2. Injektionsgerät nach Anspruch 1, gekennzeichnet durch eine Flusskontrolleinrichtung (20-25).

3. Injektionsgerät nach Anspruch 1, dadurch gekennzeichnet, dass es aus zwei Teilen (42,43) besteht, von denen einer mehrfach verwendbar und der andere wegwerfbar ist.

4. Injektionsgerät nach Anspruch 3, dadurch gekennzeichnet, dass der wegwerfbare Teil (42) den Vorratsbehälter und der wiederverwendbare Teil eine Antriebseinheit (51-54) für die Pumpeinrichtung (50) enthält.

5. Injektionsgerät nach Anspruch 1 dadurch gekennzeichnet, dass der Vorratsbehälter (3) aus einer durch eine Membran (4) abgetrennten Kammer im Gerät besteht.

6. Injektionsgerät nach Anspruch 1, dadurch gekennzeichnet, dass der Vorratsbehälter (3) aus einem Balg besteht.

7. Injektionsgerät nach Anspruch 1 dadurch gekennzeichnet, dass der Vorratsbehälter (3) aus einem Quetschschlauch besteht.

8. Injektionsgerät nach Anspruch 1, dadurch gekennzeichnet, dass der Vorratsbehälter (3) aus einer Kolbenspritze besteht, deren Zylinder als Vorratsbehälter dient.

9. Injektionsgerät nach einem der Ansprüche 6 bis 8, dadurch gekennzeichnet, dass ein saugfähiges Material zur Wirkstoffaufnahme vorgesehen ist.

10. Injektiosgerät nach Anspruch 1, dadurch gekennzeichnet, dass mehrere Vorratsbehälter vorgesehen sind.

11. Injektionsgerät nach Anspruch 1, dadurch gekennzeichnet, dass die Pumpeinrichtung aus einer von dem Vorratsbehälter durch eine elastische Membran getrennte Kammer besteht, in der Mittel zur Erzeugung eines Drucks vorgesehen sind.

12. Injektionsgerät nach Anspruch 11, dadurch gekennzeichnet, dass die Kammer mit Treibdampf gefüllt ist.

13. Injektionsgerät nach Anspruch 11, dadurch gekennzeichnet, dass in der Kammer ein elektrochemisches, photo-chemisches oder chemisches System zur Erzeugung eines Drucks vorgesehen ist.

14. Injektionsgerät nach Anspruch 11, dadurch gekennzeichnet, dass in der Kammer ein Osmose- oder Elektroosmosesystem vorgesehen ist.

15. Injektionsgerät nach Anspruch 1, dadurch gekennzeichnet, dass die Pumpeinrichtung einen mechanischen Antrieb umfasst.

16. Injektionsgerät nach Anspruch 15, dadurch gekennzeichnet, dass der mechanische Antrieb eine Feder, ein Bimetall, eine Gedächtnislegierung oder ein Uhrentriebwerk umfasst.

17. Injektionsgerät nach Anspruch 1, dadurch gekennzeichnet, dass die Pumpeinrichtung einen elektrischen oder magnetischen Antrieb umfasst.

18. Injektionsgerät nach Anspruch 17, dadurch gekennzeichnet, dass als elektrischer oder magnetischer Antrieb eine Elektropumpe, Membranpumpe, piezoelektrische Pumpe, ein elektrisches Uhrentriebwerk oder einen Elektromagneten umfasst.

19. Injektionsgerät nach Anspruch 1, dadurch gekennzeichnet, dass die Befestigungsmittel aus einer Klebschicht bestehen.

20. Injektionsgerät nach Anspruch 1, dadurch gekennzeichnet, dass die Befestigungsmittel ein Pflaster umfassen.

21. Injektionsgerät nach einem der Ansprüche 19 und 20, dadurch gekennzeichnet, dass eine Klebschicht verwendet wird, die ein Lokalanaesthetikum enthält.

22. Injektionsgerät nach Anspruch 1, dadurch gekennzeichnet, dass die Nadel einen Durchmesser von < 0,5 mm aufweist.

23. Injektionsgerät nach Anspruch 1, dadurch gekennzeichnet, dass die Nadelantriebsvorrichtung mit einem Hahn kombiniert ist, der die Verbindung zwischen Vorratsbehälter und Injektionsnadel kontrolliert.

24. Injektionsgerät nach Anspruch 16, dadurch gekennzeichnet, dass von einer Antriebsfeder nur ein Bruchteil der Federauslenkung ausgenutzt wird.

25. Injektionsgerät nach Anspruch 1, dadurch gekennzeichnet, dass eine elektronische Steuerungseinrichtung zur Steuerung der Pumpeinrichtung vorgesehen ist, wobei die Steuerungseinrichtung mit einem Datenspeicher für ein vorprogrammiertes Abgabeprofil versehen ist.

26. Injektionsgerät nach Anspruch 1, dadurch gekennzeichnet, dass als Pumpeinrichtung eine Schlauchquetschpumpe mit veränderlichem Schlauchquerschnitt vorgesehen ist, mit der das Abgabeprofil programmiert werden kann.

27. Injektionsgerät nach Anspruch 1, dadurch gekennzeichnet, dass die Nadelantriebsovorrichtung für eine Einstichtiefe von 0,5-5 mm ausgelegt ist.

28. Injektionsgerät nach Anspruch 1, dadurch gekennzeichnet, dass mit einem Septum verschlossene Oeffnungen im Vorratsbehälter vorgesehen sind, durch die dieser kurz vor dem Gebrauch aufgefüllt wird.

29. Injektionsgerät nach Anspruch 1 gekennzeichnet durch die Kombination mit Sensoren zur Steuerung oder Regelung der Abgaberate.

**Claims**

1. A portable appliance for the subcutaneous or intradermal injection of a liquid formulation of an active principle, having a supply vessel (3) for the formulation, an injection needle (7) adapted to communicate with the vessel, pump means (4, 32, 34, 50) for emptying the vessel through the injection needle and securing means (16, 17) for securing the appliance to an appropriate part of the patient's body, characterized by a needle driving means (12-13) for shooting the injection needle into the patient's skin.

2. An injection appliance according to claim 1, characterized by flow control means (20-25).

3. An injection appliance according to claim 1, characterized in that it is in two parts (42, 43), one of which is reusable and the other of which is discardable.

4. An injection appliance according to claim 3, characterized in that the discardable part (42) comprises the supply vessel and the reusable part comprises means (51-54) for driving the pump means (50).

5. An injection appliance according to claim 1, characterized in that the supply vessel (3) is a chamber in the appliance, which is separated by a diaphragm (4).

6. An injection appliance according to claim 1, characterized in that the supply vessel (3) is in the form of bellows.

7. An injection appliance according to claim 1, characterized in that the supply vessel (3) is in the form of a squeezable hose.

8. An injection appliance according to claim 1, characterized in that the supply vessel (3) is in the form of a plunger syringe whose cylinder is operative as the supply vessel.

9. An injection appliance according to any one of claims 6 to 8, characterized in that an absorbent material is provided to take up the active principle.

10. An injection appliance according to claim 1, characterized in that more than one supply vessel is provided.

11. An injection appliance according to claim 1, characterized in that the pump means comprise a chamber which is separated from the supply vessel by a resilient diaphragm and in which pressure-producing means are provided.

12. An injection appliance according to claim 11, characterized in that the chamber is filled with propellant vapour.

13. An injection appliance according to claim 11, characterized in that an electrochemical, photochemical or chemical system for producing a pressure is provided in the chamber.

14. An injection appliance according to claim 11, characterized in that an osmosis or electro-osmosis system is provided in the chamber.

15. An injection appliance according to claim 1, characterized in that the pump means comprise a mechanical drive.

16. An injection appliance according to claim 15, characterized in that the mechanical drive comprises a spring, a bimetal, a memory alloy or a clockwork drive.

17. An injection appliance according to claim 1, characterized in that the pump means comprise an electric or magnetic drive.

18. An injection appliance according to claim 17, characterized in that the electric or magnetic drive comprises an electric pump, a diaphragm pump, a piezoelectric pump, an electric clock drive or an electromagnet.

19. An injection appliance according to claim 1, characterized in that the securing means are in the form of an adhesive layer.

20. An injection appliance according to claim 1, characterized in that the securing means comprise a plaster.

21. An injection appliance according to either of claims 19 and 20, characterized in that an adhesive layer containing a local anaesthetic is used.

22. An injection appliance according to claim 1, characterized in that the needle has a diameter of < 0.5 mm.

23. An injection appliance according to claim 1, characterized in that the needle driving means are combined with a valve which controls communication between the supply vessel and the injection needle.

24. An injection appliance according to claim 16, characterized in that only a fraction of the spring deflection of a driving spring is used.

25. An injection appliance according to claim 1, characterized in that electronic control means for controlling the pump means are provided, the control means having a data memory for a programmed release profile.

26. An injection appliance according to claim 1, characterized in that the pump means are in the form of a squeezable hose pump which is of variable hose cross-section and which enables the release programme to be programmed.

27. An injection appliance according to claim 1, characterized in that the needle driving means are designed for a penetration depth of 0.5-5 mm.

28. An injection appliance according to claim 1, characterized in that the vessel is provided with apertures which are closed by a partition through which the vessel is filled shortly before use.

29. An injection appliance according to claim 1, characterized by the combination with sensors to control or vary the release rate.

**Revendications**

1. Appareil portatif pour l'injection sous-cutanée ou intradermique d'une formulation de substance active liquide, comprenant un réservoir (3) de la formulation de substance active, une aiguille d'injection (7) pouvant se raccorder au réservoir, un dispositif de pompage (4, 32, 34,50) destiné a vider le réservoir par l'aiguille d'injection et des organes (16,17) de fixation de l'appareil en un emplacement convenable du corps du patient, caractérisé' par un dispositif de commande de l'aiguille (12-13) destiné à insérer l'aiguille d'injection dans la peau du patient.

2. Appareil d'injection selon la revendication 1, caractérisé par un dispositif de contrôle du flux (20-25).

3. Appareil d'injection selon la revendication 1, caractérisé en ce qu'il est en deux parties (42, 43) dont l'une est utilisable plusieurs fois et l'autre est jetable.

4. Appareil d'injection selon la revendication 3, caractérisé en ce que la partie jetable (42) comprend le réservoir et la partie réutilisable comprend une unité (51-54) de commande du dispositif de pompage (50).

5. Appareil d'injection selon la revendication 1, caractérisé en ce que le réservoir (3) consiste en une chambre située dans l'appareil et isolée par une membrane (4).

6. Appareil d'injection selon la revendication 1, caractérisé en ce que le réservoir (3) consiste en un soufflet.

7. Appareil d'injection selon la revendication 1, caractérisé en ce que le réservoir consiste en un tube souple.

8. Appareil d'injection selon la revendication 1, caractérisé en ce que le réservoir (3) consiste en une seringue à piston dont le cylindre sert de réservoir.

9. Appareil d'injection selon l'une des revendications 6 à 8, caractérisé en ce qu'une matière absorbante est prévue pour recueillir la substance active.

10. Appareil d'injection selon la revendication 1, caractérisé en ce que plusieurs réservoirs sont prévus.

11. Appareil d'injection selon la revendication 1, caractérisé en ce que le dispositif de pompage se compose d'une chambre séparée du réservoir par une membrane élastique et dans laquelle sont prévus des moyens pour produire une pression.

12. Appareil d'injection selon la revendication 11, caractérisé en ce que la chambre est remplie de vapeur de propulsion.

13. Appareil d'injection selon la revendication 11, caractérisé en ce qu'un système électrochimique, photochimique ou chimique de production d'une pression est prévu dans la chambre.

14. Appareil d'injection selon la revendication 11, caractérisé en ce qu'un système d'osmose ou d'électro-osmose est prévu dans la chambre.

15. Appareil d'injection selon la revendication 11, caractérisé en ce que le dispositif de pompage comprend une commande mécanique.

16. Appareil d'injection selon revendication 15, caractérisé en ce que la commande mécanique est un ressort, une bilame, un alliage à mémoire ou un mouvement d'horloge.

17. Appareil d'injection selon la revendication 1, caractérisé en ce que le dispositif de pompage comprend une commande électrique ou magnétique.

18. Appareil d'injection selon la revendication 17, caractérisé en ce que la commande électrique ou magnétique consiste en une électropompe, une pompe à diaphragme, une pompe piezoélectrique, un mouvement électrique d'horloge ou un électro-aiment.

19. Appareil d'injection selon la revendication 1, caractérisé en ce que les organes de fixation consistent en une couche d'adhésif.

20. Appareil d'injection selon la revendication 1, caractérisé en ce que les organes de fixation comprennent un emplâtre.

21. Appareil d'injection selon l'une des revendications 19 et 20, caractérisé en ce qu'une couche d'adhésif qui est utilisée contient un anesthésique locale.

22. Appareil d'injection selon la revendication 1, caractérisé en ce que l'aiguille à un diamètre de < 0,5mm.

23. Appareil d'injection selon la revendication 1, caractérisé en ce que le dispositif de commande de l'aiguille est combiné avec un robinet qui contrôle la liaison entre le réservoir et l'aiguille d'injection.

24. Appareil d'injection selon la revendication 16, caractérisé en ce que seule une fraction de la détente élastique d'un ressort de commande est utilisée.

25. Appareil d'injection selon la revendication 1, caractérisé en ce qu'un dispositif électronique de commande du dispositif de pompage est prévu, le dispositif de commande étant équipé d'une mémoire de données pour un profil de distribution préalablement programmé.

26. Appareil d'injection selon la revendication 1, caractérisé en ce que le dispositif de pompage qui est prévu est une pompe péristaltique dont la section du tuyau est variable et permet de programmer le profil de distribution.

27. Appareil d'injection selon la revendication 1, caractérisé en ce que le dispositif de commande de l'aiguille est conçu pour une profondeur d'insertion de 0,5-5mm.

28. Appareil d'injection selon la revendication 1, caractérisé en ce que le réservoir comporte intérieurement des ouvertures obturées par un septum et par lesquelles il est rempli peu avant l'utilisation.

29. Appareil d'injection selon la revendication 1, caractérisé par la combinaison avec des détecteurs de commande ou de réglage du taux de distribution.

Fig.1

Fig. 2

Schnitt A-A

Fig. 3

Schnitt B-B

Fig. 4

Schnitt A-A

Fig. 5

Schnitt B-B

Fig. 6

Fig. 7

Schnitt A-A

Fig. 8

Fig. 9